# DEMANDE DE BREVET EUROPEEN

(11) **EP 4 513 277 A1**
(43) Date de publication de la demande: **26.02.2025**
(21) Numéro de dépôt: 23192727.8
(22) Date de dépôt: 22.08.2023
(51) Int. Cl.: G04B 37/04, G04B 47/06

(54) **SUPPORT POUR MONTRE AMOVIBLE**

(71) Demandeur: Aima Innovation Sàrl, 2900 Porrentruy (CH)
(72) Inventeur: Baute, Victor, 68720 Spechbach (FR)
(74) Mandataire: P&TS SA (AG, Ltd.)

(57) **Abrégé**

La présente invention concerne un support (20) comprenant une partie périphérique (21) et une surface interne (200) délimitant un espace ajouré (25) et comprenant un rebord interne (22) formant un premier méplat (220), et un dispositif de verrouillage (240) adapté pour fixer un objet (10) de manière amovible. Le support de la présente invention comprend en outre des surfaces de guidage propres à guider un applicateur de capteur physiologique, pouvant être masqué par l'objet auquel il est assemblé. L'invention se réfère en outre à une méthode d'application d'un capteur physiologique à l'aide d'un applicateur.

## Description

### Domaine technique

La présente invention concerne un support adapté pour recevoir de manière amovible une pièce d'horlogerie telle qu'une montre. L'invention concerne également une pièce d'horlogerie comprenant un dispositif de fixation amovible lui permettant de se fixer sur un tel support. Elle concerne en outre la combinaison d'un tel support avec une telle pièce d'horlogerie. Le support selon la présente invention est en outre adapté au centrage ou au guidage d'un dispositif d'application de capteur médical tel qu'un capteur de glycémie. La présente invention porte en outre sur une méthode d'implantation d'un capteur physiologique à l'aide d'un tel support.

### Etat de la technique

Des capteurs de glycémie tels que ceux développés par Abbott peuvent être installés directement sur la peau du patient et permettre ainsi une surveillance continue de la glycémie. De tels capteurs se présentent souvent sous la forme d'une pastille comprenant sur sa face antérieure un filament souple, lequel peut s'insérer sous la peau pour capter les paramètres désirés. L'installation adéquate d'un tel capteur nécessite l'utilisation d'un applicateur par l'utilisateur ou le patient, qui peut ainsi appliquer le capteur de manière autonome. Le positionnement de l'applicateur sur soi-même n'est cependant pas toujours aisé. La position finale du capteur est alors approximative. L'applicateur est conçu pour poser le capteur sur une partie discrète du patient, telle que l'arrière du haut d'un bras, ce qui nécessite une certaine souplesse de la part de l'utilisateur. En fonction des personnes, l'opération peut se révéler difficile ou délicate. De surcroît, le capteur peut rester visible par les tiers.

Par ailleurs, dans le domaine de l'horlogerie ou de la bijouterie, il peut être attrayant de varier les apparences esthétiques des objets portés par un utilisateur sans avoir à changer l'intégralité du bijou ou de la pièce d'horlogerie.

Il y a donc matière à développer un système qui permette l'implantation simple d'un capteur physiologique tel qu'un capteur de glycémie, à l'aide d'un applicateur, et qui permette en outre de dissimuler un tel capteur. Il y a en outre matière à proposer un système qui permette de varier l'apparence esthétique d'un ornement porté par un utilisateur.

### Bref résumé de l'invention

Un but de la présente invention est de proposer un dispositif permettant de fixer de manière amovible une pièce d'horlogerie ou un bijou ou tout autre objet destiné à être porté par un utilisateur. En l'occurrence, un objectif de la présente invention est de proposer un dispositif adapté à maintenir un objet à distance de la peau de l'utilisateur.

Un autre objectif est de proposer un dispositif adapté à masquer un capteur physiologique ou un patch ou tout équivalent.

Un autre objectif de la présente invention est de proposer un guide ou un système de positionnement d'un applicateur de capteur physiologique.

Un autre but de la présente invention est de proposer une méthode d'application d'un capteur physiologique à l'aide d'un applicateur.

Selon l'invention, ces buts sont atteints, notamment au moyen de l'invention objet des revendications indépendantes et détaillé dans les revendications qui en dépendent.

Cette solution présente notamment l'avantage par rapport à l'art antérieur de cumuler plusieurs fonctions parmi celles de masquer un capteur physiologique, de simplifier l'application d'un capteur physiologique et de varier l'aspect esthétique d'un ornement. D'autres avantages apparaitrons dans la description détaillée ci-dessous.

### Brève description des figures

Des exemples de mise en oeuvre de l'invention sont indiqués dans la description illustrée par les figures suivantes :
- Figure 1 : vue en perspective d'un support selon un mode de réalisation de la présente invention,
- Figures 2a, 2b : détails schématique d'un dispositif de verrouillage selon deux modes de réalisations de la présente invention.
- Figure 3 : représentation schématique d'un ensemble comprenant un support selon la présente invention, d'un applicateur de l'art antérieur et d'un capteur de l'art antérieur,
- Figure 4 : Représentation schématique d'un ensemble comprenant un support et un objet adapté, selon un mode de réalisation de la présente invention,
- Figure 5 : Détails d'un support vue en perspective, selon un mode de réalisation de la présente invention,

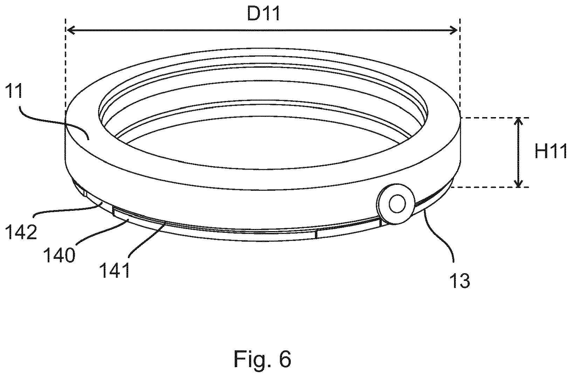

- Figure 6 : Détails d'un objet pouvant être fixé sur le support selon un mode de réalisation de la présente invention,
- Figure 7 : Vue éclatée d'un ensemble comprenant un support et un objet selon un mode de réalisation de la présente invention.

### Exemple(s) de mode de réalisation de l'invention

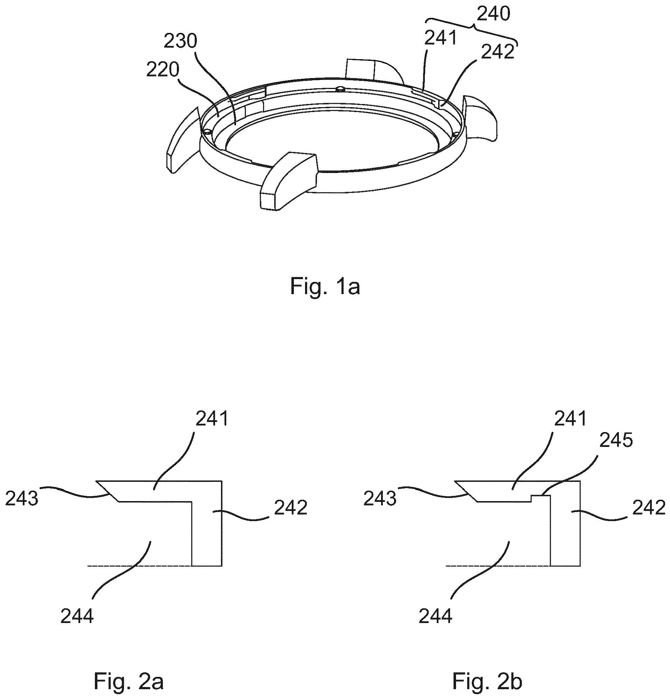

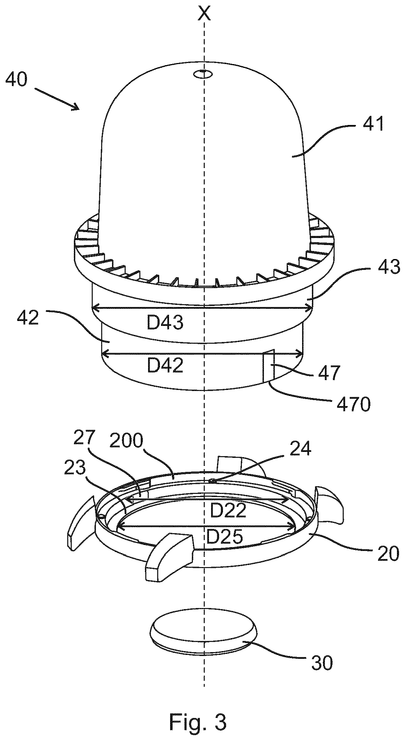

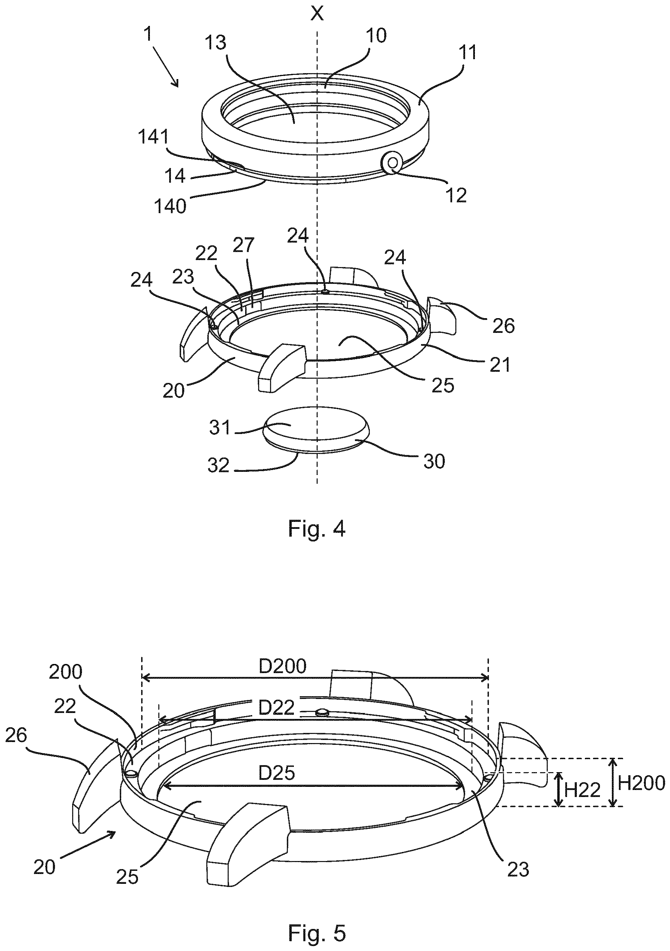

La présente description porte sur un support 20 tel qu'illustré par les figures 1, 3 et 4. Le support 20 comporte une partie périphérique 21 délimitant un espace ajouré 25. La partie périphérique peut prendre toute forme adéquate. Elle peut être par exemple de forme circulaire, ovale, carrée, rectangulaire ou mixte. La partie périphérique **21** comprend une surface interne **200,** orientée vers l'espace ajouré **25.** La surface interne **200** comporte un rebord interne **22** formant un premier méplat **220.** Selon un mode de réalisation préféré, le premier méplat **220** est orthogonal à la surface interne **200** du support. En particulier, la surface interne **200** peut être orientée verticalement et le premier méplat **220** horizontalement.

Le premier méplat **220** permet d'accueillir la surface inférieure **13** d'un objet **10** à fixer sur le support **20.** Cela représente en outre l'avantage de laisser un espace libre sous la surface inférieure **13** de l'objet **10** lorsqu'il est porté par un utilisateur. L'espace libre est fonction de la hauteur **H22** du rebord interne **22.** Elle est typiquement comprise entre 1 millimètre et 10 millimètres, ou de l'ordre de 2 à 8 millimètres. La surface inférieure **13** de l'objet **10** est ainsi à distance de la peau de l'utilisateur, ce qui limite la transpiration. A cet effet, la partie périphérique **21** du support peut être plein ou bien ajourée de trous d'aération (non représentés) laissant circuler l'air sous la surface inférieure **13** de l'objet **10.**

La surface interne **200** du support **20** a une dimension **D200** adaptée à la dimension **D11** de la structure externe de l'objet **10** à assembler au support **20.** En d'autres termes, la dimension **D200** de la surface interne **200** correspond à la dimension **D11** de la structure externe **11** de l'objet **10** aux jeux mécaniques près. De la sorte, l'objet **10** peut être inséré dans l'espace délimité par la surface interne **200** au moins sur une portion de sa hauteur **H11** de sorte à reposer sur le premier méplat **220.** A cet effet la dimension **D22** du rebord interne **22** est inférieure à la dimension **D11** de la structure externe **11** de l'objet **10** à assembler sur le support **20.** La hauteur **H22** du rebord interne **22** représente une fraction de la hauteur **H200** de la surface interne **200.** La hauteur **H22** du rebord interne peut être comprise entre 10% et 90% de la hauteur **H200** de la surface interne **200**, en particulier de l'ordre de 20%, 30%, 50%, 60% ou 70%.

Le support **20** comprend avantageusement une lèvre inférieure **23** disposée sur la partie inférieure de la structure périphérique **21.** La lèvre inférieure **23** délimite un espace ajouré **25** de dimension **D25.** Le support **20** est ainsi ajouré de sorte qu'une fois disposé sur l'utilisateur, sa peau reste accessible à travers la partie centrale du support **20.** La dimension **D25** de l'espace ajouré **25** représente une fraction comprise entre 50% et 95%, de préférence entre 70% et 85%, soit de l'ordre de 75% ou 80%, de la dimension **D200** de la surface interne **200.** La lèvre **23** forme un second méplat **230.**

Le support **20** comporte au moins un dispositif de maintien **26,** permettant de le disposer sur l'utilisateur. Selon un mode de réalisation préféré, le dispositif de maintien **26** comporte deux paires de cornes adaptées à la fixation d'un bracelet (non représenté) permettant de fixer le support **20** sur la poignet de l'utilisateur. D'autres dispositifs de maintien peuvent néanmoins être utilisés selon les besoins.

Le support **20** selon la présente description comporte un dispositif de verrouillage **240** permettant de maintenir de manière amovible l'objet **10** à assembler sur le support **20.** Tout dispositif de verrouillage adéquat peut être envisagé tel qu'un pas de vis, un système de baïonnette, un ou plusieurs ergots rétractables, un système de clips impliquant par exemple une lame de ressort, ou leur combinaison, ainsi que tout équivalent.

Selon un mode de réalisation, le dispositif de verrouillage **240** s'apparente à un système de baïonnette, illustré par les figures 1, 2a et 2b. Il comprend à cet effet une, de préférence deux ou plus de deux, lèvres de verrouillage **241** disposée sur une fraction de la surface interne **200** du support **20.** La ou les lèvres de verrouillage **241** sont disposées parallèlement au premier méplat **220** de sorte à former une glissière de verrouillage **244** délimitée par le premier méplat **220.** Une autre surface que le premier méplat peut néanmoins être utilisée à cette fin. La ou les lèvres de verrouillage **241** comportent une butée de verrouillage **242** à l'une de leur extrémité, fermant au moins partiellement la glissière de verrouillage **244.** Selon un mode de réalisation, une lèvre de verrouillage selon la présente description peut comprendre un biseau **243** orienté vers la glissière de verrouillage **244** (figure 2a, 2b). Alternativement ou en plus, une lèvre de verrouillage selon la présente invention peut comporter un cran de verrouillage **245** (figure 2b) dans lequel peut s'insérer un ergot ou une pièce équivalente de l'objet **10** à assembler au support **20.**

L'objet **10** à assembler au support **20** comporte une structure externe **11** dont la dimension **D11** est adaptée au support **20** ici décrit. La structure externe **11** peut comprendre une surface inférieure **13** délimitant la partie inférieure de l'objet **10.** La structure externe **11** comporte en outre un dispositif d'ancrage adapté pour coopérer avec le dispositif de verrouillage **240** du support **20.** Dans le cas d'un pas de vis, la structure externe **13** comporte un pas de vis complémentaire. Dans le cas d'un système de type baïonnette, la structure externe **11** comporte au moins un ergot ou une lèvre pouvant coopérer avec la ou les glissières de verrouillage du support **20.** Dans le cas d'un système de clipsage à ressort, l'objet **10** comporte le relief adapté pour recevoir et maintenir la lame de ressort.

Selon un mode de réalisation préféré, la structure externe **11** de l'objet **10** comporte en position inférieure un socle d'ancrage **140.** Le socle d'ancrage **140** s'étend sur une fraction de la périphérie de l'objet **10** jusqu'à une surface d'emboitement **142.** Il peut prendre la forme d'une lèvre ou d'un rebord en périphérie de la structure externe **11** adaptée pour s'insérer dans la glissière de verrouillage **244** du support **20.** Alternativement, le socle d'ancrage **140** peut se limiter à un ergot dont le diamètre correspond à la largeur de la glissière de verrouillage **144.** Un tel ergot peut en outre s'insérer dans un cran de verrouillage **245** ménagé dans la lèvre de verrouillage **241,** par exemple en fin de glissière de verrouillage **244.** De préférence, plusieurs socles d'ancrage **140** sont disposés sur le pourtour de la structure externe **11,** soit deux ou plus de deux. Ils sont séparés les uns des autres par des surfaces d'emboitement **142** permettant le passage des lèvres de verrouillage **241** du support **20.** Le dispositif d'ancrage de l'objet 10 comporte en outre une rainure d'ancrage **141** permettant le glissement de la lèvre de verrouillage **241** du support **20.** De la sorte, l'utilisateur peut disposer l'objet **10** sur le support **20,** en faisant coïncider les surfaces d'emboitement **142** avec les lèvres de verrouillage **241** du support **20** jusqu'à faire reposer sa surface inférieure **13** sur le premier méplat **220,** puis exercer une rotation d'un angle adapté pour insérer le ou les socles d'ancrage **140** dans les glissières de verrouillage **244** correspondantes jusqu'à leur butée de verrouillage **242.** L'objet **10** est ainsi sécurisé sur le support **20.**

La largeur des glissières des verrouillage **244** et les socles d'ancrage **141** peuvent être ajustés de sorte que les frottements soient suffisants pour maintenir en place l'objet **10.**

Alternativement, un dispositif élastique permettant d'augmenter les frottement, telle qu'une lame de ressort, peut être prévu.

Selon un mode de réalisation, le support **20** peut comporter, en complément du dispositif de verrouillage ici-décrit, un système de blocage **24** permettant de mieux sécuriser l'objet **10** sur le support **20.** Un tel système de blocage peut comporter par exemple une ou plusieurs billes ou ergots rétractables disposés sur une surface du support **20.** De telles billes ou ergots rétractables peuvent ainsi s'effacer dans le support **20** au contact de l'objet **10** et en ressortir automatiquement en vis-à-vis d'une cavité de l'objet **10** de sorte à la bloquer en position. Une telle bille ou ergot rétractable est associée à un ressort adapté à maintenir la bille ou l'ergot en position proéminant par rapport à la surface du support **20.** Une telle bille ou ergot est généralement connue désigné par le terme de bille à cliquet.

Selon un mode de réalisation, le premier méplat **220** est pourvu d'une ou plusieurs de ces billes ou ergots rétractables **24.** La surface inférieure **13** de l'objet **10** est quant à lui pourvu de cavités adaptées à accueillir de telles billes ou ergots rétractable lorsque la surface inférieure **13** repose sur le premier méplat **220.**

Le système de blocage **24** peut être prévu en complément des butées de verrouillage **242** ou bien en remplacement des butées de verrouillage **242.** Les billes rétractables peuvent être disposées ailleurs que sur le premier méplat **220,** par exemple, sur la surface interne **200,** dans ce cas, les cavités correspondantes sont disposées en vis-à-vis sur la structure externe **11** de l'objet **10.** Selon un mode de réalisation, plusieurs billes ou ergots rétractable sont disposés de manière symétrique de sorte que l'objet **10** peut être fixé sur le support **20** selon différentes orientations. Alternativement, les billes ou ergots rétractables sont disposés de façon non symétrique de sorte à servir de détrompeur. L'objet **10** est ainsi assemblé au support **20** selon une seule orientation.

Selon un mode de réalisation, les lèvres de verrouillages **241** et les surface d'emboitement **142** correspondantes sont disposées de manière symétrique de sorte que l'objet **10** puisse être assemblé au support selon plusieurs argentations angulaire différentes. Alternativement, les lèvres de verrouillage **241** et les surfaces d'emboitement **142** correspondantes sont disposées de manière non symétrique de sorte à servir de détrompeur.

Le nombre de lèvres de verrouillage **241** dans un dispositif de verrouillage **240** peut varier selon les besoins. Elles peuvent être par exemple au nombre de deux ou trois ou quatre ou plus de quatre.

Le nombre de billes ou ergots rétractables du système de blocage **24** peut également varier selon les besoins. Selon une option, aucun système de blocage n'est prévu. Selon une option préférée, le système de blocage comprend une ou deux ou trois ou quatre billes ou ergots rétractables. Selon une variante, la disposition des billes ou ergots rétractables du système de blocage **24** est inversée, de sorte que le premier méplat **220** comporte des cavités et que l'objet **10** comporte de telles billes ou ergots rétractables.

Bien que le dispositif d'ancrage **14** de l'objet et le dispositif de verrouillage **240** soient ici décrits sur la base d'un système de baïonnette, d'autres combinaisons sont bien entendu possibles. Par exemple, des filets obliques peuvent remplacer les lèvres de verrouillage **241** sur la surface interne **200** et le socle d'ancrage peut prendre la forme de filets obliques complémentaires, de sorte à produire un système de quart de tour ou de tiers de tour.

L'objet **10** peut désigner tout type d'objet d'ornement ou d'utilité. En l'occurrence la structure externe **11** peut servir de boitier dans lequel un bijou ou un mouvement horloger peut être logé.

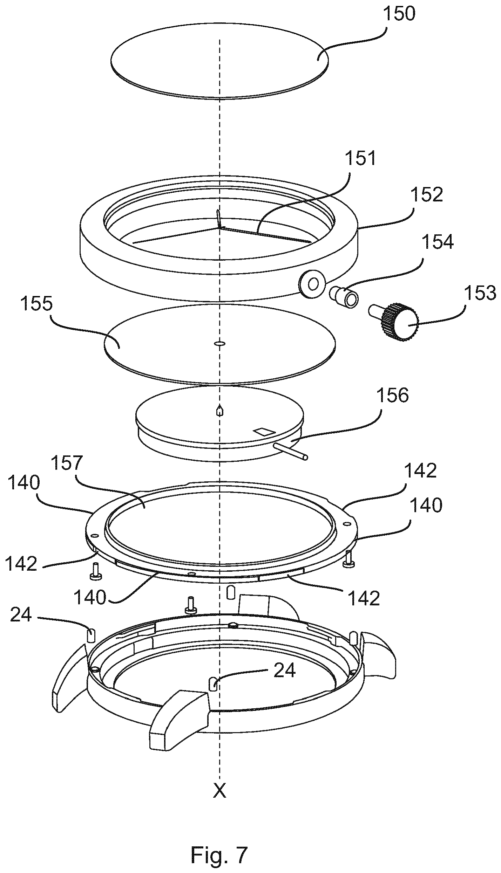

Selon un mode de réalisation, l'objet **10,** illustré plus en détail par la figure 7, comprend un calibre **156,** un cadran **155** surmonté d'aiguilles **151** et d'un verre **150** et logé dans la carrure **152** qui fait office de structure externe **11.** Le fond **157,** comprenant une surface inférieure **13,** comporte également le dispositif d'ancrage **14.** Le fond peut par exemple être vissé sur la carrure ou assemblé de toute autre manière adéquate.

L'invention selon la présente description couvre un ensemble modulable comprenant le support **20** ici décrit et un objet **10** assemblé de manière amovible au support **20.** Selon les besoins, différents objets **10** peuvent être assemblés au support **20.** Notamment, un objet **10** peut être sélectionné parmi un cadran à affichage digital ou numérique, un cadran à affichage mécanique, un bijou, ou une combinaison de plusieurs objets.

La présente invention couvre également un kit ou un set comprenant un support **20** comme décrit ici et plusieurs objets **10** différents pouvant être alternativement assemblés au support **20** de manière amovible.

Alternativement ou en plus, le kit comprend, outre le support **20,** un ou plusieurs capteurs **30** et un ou plusieurs applicateurs **40** adaptés à disposer le capteur **30** sur l'utilisateur.

De préférence, le support **20** ici-décrit possède plusieurs fonctions. La première est celle de maintenir un objet **10** sur l'utilisateur de façon amovible. Une autre fonction est de maintenir sur l'utilisateur un objet **10** tout en laissant un espace libre entre l'objet **10** et la peau de l'utilisateur. L'espace libre est notamment possible grâce au rebord intérieur **22,** d'une hauteur **D22,** décrit plus haut. L'espace libre peut être utilisé pour masquer la présence d'un capteur **30** ou d'un patch ou de tout autre dispositif à usage médical ou pour d'autres usages, sur la peau de l'utilisateur. La dimension **D25** de l'espace ajouré **25** est ainsi prévue à cet effet en fonction de la dimension du patch ou du capteur à placer sur l'utilisateur.

Selon un mode de réalisation, le support **20** sert en outre de guide ou de cadre de centrage pour l'application d'un capteur **30** sur la peau de l'utilisateur. A cet effet, le support **20** peut comporter une ou plusieurs surface de guidage **27** disposées sur la surface interne **200.** Une surface de guidage **27** selon la présente description peut consister en un relief rectiligne orienté transversalement à la surface interne **200,** c'est-à-dire verticalement lorsque le support **20** est disposé sur l'utilisateur. Le relief de la surface de guidage **27** peut comprendre ou consister en une rainure, une lèvre, un ergot, ou tout équivalent. Une telle surface de guidage s'inscrit donc en creux ou en surépaisseur par rapport à la surface interne **200.** Elle traverse en outre le premier méplat **220** au moins jusqu'à la lèvre inférieure **23.**

Le support **20** est ainsi compatible avec l'utilisation d'un applicateur **40** comprenant une partie préhensile **41,** une base **43** et un socle d'applicateur **42.** Selon un mode de réalisation, le socle d'applicateur **42** peut comporte un ou plusieurs reliefs complémentaires des surfaces de guidage **27** du support **20.** L'applicateur **40** peut être disposé dans le support **20** et guidé par la ou les surfaces de guidage **27** du support jusqu'à venir en appui sur la lèvre inférieure **23.**

Selon un mode de réalisation, le support **20** est exempt de lèvre inférieure **23** de sorte que l'applicateur vienne directement au contact de la peau de l'utilisateur. L'actionnement de l'applicateur permet ensuite de fixer le capteur **30** sur la peau de l'utilisateur d'une manière précise et reproductible. Le capteur **30** est alors placé en position centrale de l'espace ajouré **25.**

Le socle **43** a une dimension de socle **43** correspondant à la dimension de rebord interne **D22** du support **20,** aux jeux fonctionnels près.

La présente invention couvre en outre une méthode de positionnement d'un capteur **30** ou d'un implant ou tout équivalent sur un utilisateur. Pour ce faire, la méthode comporte une première étape de placer et fixer le support **20** sur l'utilisateur, par exemple à l'un de ses poignets. Le dispositif de maintien **26** est particulièrement adapté pour cette opération. Une fois le support **20** placé sur l'utilisateur, celui-ci peut préparer l'applicateur **40** avec le capteur **30** approprié et mettre l'applicateur **40** en contact avec le support **20** de sorte que les reliefs présents au niveau du socle de l'applicateur complémentent la ou les surfaces de guidage **27.** L'utilisateur exerce ensuite la pression nécessaire sur l'applicateur pour fixer le capteur **30** correctement. La méthode comporte une étape de fixer sur le support un objet **10** de sorte que le capteur **30** ne soit plus visible par un tiers.

Selon un mode de réalisation particulier, l'objet **10** comporte un module d'affichage de données médicales ou physiologiques telles que celles mesurées par le capteur **30.** Le capteur **30** peut être pourvu à cet effet d'un système de communication des données adapté.

Le capteur **30** désigne tout dispositif de détection et/ou de mesure et de transmission de données physiologiques telles que la glycémie, ou d'autres paramètres physiologiques nécessitant un suivi permanant. Un capteur **30** peut par exemple comprendre une pastille pourvue d'un ou plusieurs filaments pouvant être insérés sous la peau. Un capteur peu être conçu pour détecter et/ou mesure d'autres paramètres tels que pression artérielle, rythme cardiaque, taux d'oxygénation sanguine, que ce soit à des fins médicales ou d'entrainement sportif. Un capteur peut en outre désigner un implant ou tout autre dispositif similaire. Un capteur peut de surcroit comprendre un dispositif de traitement tel qu'un système de diffusion ou d'injection de produit selon les données collectées.

Pour les besoins de la présente description, les termes « inférieur », « supérieur », « vertical », « horizontal » ainsi que les termes relatifs s'entendent selon leur sens communément admis. En l'occurrence, ces termes s'appliquent au support **20** dans son utilisation courante. Lorsqu'il est disposé sur l'utilisateur ou posé sur une surface, la surface intérieure **200** représente la verticale, la lèvre inférieure représente l'horizontale. La partie du support **20** en contact avec l'utilisateur est en position inférieure.

Les dimensions d'espace ajouré **D25,** de rebord interne **D22,** de surface interne **D200,** de base **D42,** de socle **D43** se comprennent comme désignant la dimension caractéristique de l'élément considéré. Selon un mode de réalisation, le support **20** est de forme circulaire, les dimension correspondantes désignent des diamètres.

### Numéros de référence employés sur les figures

- 1: Système modulaire
- 10: Objet supporté
- 11: Structure externe
- D11: Dimension de structure externe
- H11: Hauteur de structure externe
- 12: Dispositif de commande
- 13: Face inférieure
- 14: Dispositif d'ancrage
- 140: Socle d'ancrage
- 141: Rainure d'ancrage
- 142: Surface d'emboitement
- 150: Verre
- 151: Aiguilles
- 152: Carrure
- 154: Tube
- 153: Couronne
- 155: Cadran
- 156: Calibre
- 157: Fond
- 20: Support
- 200: Surface interne
- D200: Dimension de surface interne
- 21: Structure périphérique
- 22: Rebord interne
- H22: Hauteur de rebord interne
- 220: Premier méplat
- D22: Dimension de rebord interne
- 23: Lèvre inférieure
- 230: Second méplat
- 24: Dispositif de blocage
- 240: dispositif de verrouillage
- 241: Lèvre de verrouillage
- 242: Butée de verrouillage
- 243: Chanfrein
- 244: Glissière de verrouillage
- 245: Cran de verrouillage
- 25: Espace ajouré
- D25: Dimension d'espace ajouré
- 26: Dispositif de maintien
- 27: Surface de guidage
- 30: Capteur
- 31: Face supérieure de capteur
- 32: Face inférieure de capteur
- 40: Applicateur
- 41: Partie préhensile
- 42: Base d'applicateur
- D42: Dimension de base
- 43: Socle d'applicateur
- D43: Dimension de socle

## Revendications

1. Support (20) destiné à recevoir un objet (10) comprenant une structure externe (11) et un dispositif d'ancrage (14), ledit support (20) comprenant :
- une partie périphérique (21) adaptée pour accueillir ladite structure externe (11),
- un dispositif de maintien (26) adapté à maintenir ledit support sur l'utilisateur, et
- une surface interne (200) de dimension (D200),
caractérisé en ce ladite surface interne (200) délimite un espace ajouré (25) de dimension d'espace ajouré (D25), qu'elle comprend un rebord interne (22) de dimension (D22) et de hauteur (H22) formant un premier méplat (220), et en ce qu'elle comprend un dispositif de verrouillage (240) adapté à coopérer avec ledit dispositif d'ancrage dudit objet (10) de sorte à le fixer de manière amovible sur le support (20).

2. Support selon la revendication 1, ledit dispositif de verrouillage comprenant (240) une ou plusieurs lèvres de verrouillage (241) orientée parallèlement audit rebord interne (22) et une butée de verrouillage (242) orientée transversalement à la surface interne (200).

3. Support selon la revendication 2, ladite une ou plusieurs lèvres de verrouillage comprenant une surface biseautée (243).

4. Support selon l'une des revendications 1 à 3, ledit support (20) comprenant en outre un dispositif de blocage (24) rétractable.

5. Support selon la revendication 4, ledit dispositif de blocage (24) comprenant une ou plusieurs billes ou ergots rétractables.

6. Support selon l'une des revendications 1 à 5, comprenant en outre une ou plusieurs surfaces de guidage (27) orientées transversalement à la surface interne (200).

7. Support selon la revendication 6, lesdites une ou plusieurs surfaces de guidage (27) désignant une rainure, un ergot ou une lèvre de guidage.

8. Support selon l'une des revendications 1 à 7, comprenant en outre une lèvre inférieure (23) formant un second méplat (D23).

9. Support selon l'une de revendications 1 à 8, ledit dispositif d'ancrage (14) dudit objet (10) comprenant un ou plusieurs socles d'ancrage (140) disposés en partie inférieure de la structure externe (11) et délimités par au moins une surface d'emboitement (142) et une ou plusieurs rainures d'ancrage (141) surmontant lesdits un ou plusieurs socles d'ancrage (140).

10. Support selon l'une des revendications 1 à 9, ledit objet (10) comprenant une surface inférieure (13), ledit premier méplat (220) étant adapté à être en contact avec ladite surface inférieure (13).

11. Kit comprenant un support (20) selon l'une des revendications 1 à 10, et deux ou plus de deux objets (10) différents sélectionnés parmi une pièce horlogère à affichage mécanique, une pièce horlogère à affichage digital, un bijou, un module d'affichage de données médicales ou physiologiques.

12. Kit selon la revendications 11, comprenant en outre un applicateur (40) de capteur (30) et au moins un capteur (30) de données physiologiques ou médicales, ledit applicateur comprenant un ou plusieurs reliefs de guidage complémentaires des surfaces de guidage (27) dudit support (20).

13. Méthode d'application d'un capteur (30) de données physiologiques comprenant les étapes
- d'associer ledit capteur (30) à un applicateur (40) comprenant un ou plusieurs reliefs de guidage,
- disposer le support selon l'une des revendications 6 à 10 sur un utilisateur au moyen de son dispositif de maintien (26),
- associer ledit applicateur audit support (20), de sorte que lesdites une ou plusieurs surfaces de guidage (27) coopèrent avec lesdits un ou plusieurs reliefs de guidage de l'applicateur, et
- appliquer le capteur (30) en actionnant ledit applicateur (40).

14. Méthode selon la revendication 13, comprenant en outre une étape d'assembler de manière amovible un objet (10) sur ledit support (20).

15. Objet (10) adapté à être assemblé de manière amovible sur un support selon l'une des revendication 1 à 10, ledit objet comprenant un fond (157), une carrure (152) associée au fond (157) et comprenant un calibre (156), un écran (155), des aiguilles (151) et un verre (150), **caractérisé en ce que** le fond est pourvu d'un ou plusieurs socles d'ancrage (140) et d'une ou plusieurs surfaces d'emboitement (142).
